# EUROPEAN PATENT SPECIFICATION

(11) **EP 2 877 447 B1**
(45) Date of publication and mention of the grant of the patent: **15.06.2016**
(21) Application number: 13734729.0
(22) Date of filing: 03.07.2013
(51) Int. Cl.: C07C 273/10, B01J 19/00

(54) **USE OF UREA SYNTHESIS PURGE GAS IN AN INTEGRATED AMMONIA-UREA PROCESS AND RELATED PLANT**
VERWENDUNG VON HARNSTOFFSYNTHESE-SPÜLGAS IN EINEM INTEGRIERTEN AMMONIUM-HARNSTOFFVERFAHREN UND ZUGEHÖRIGE ANLAGE
UTILISATION DE GAZ DE PURGE DE SYNTHÈSE D'URÉE DANS UN PROCÉDÉ INTÉGRÉ DE PRODUCTION D'URÉE-AMMONIAC ET INSTALLATION ASSOCIÉE.

(30) Priority: 25.07.2012 EP 12177783
(43) Date of publication of application: 03.06.2015
(73) Proprietor: Casale SA, 6900 Lugano-Besso (CH)
(72) Inventor: ZARDI, Federico, 6932 Breganzona (CH)
(74) Representative: Zardi, Marco
(86) International application number: PCT/EP2013/064035
(87) International publication number: WO 2014/016089

(56) References cited:
- EP-A1- 0 748 794
- EP-A1- 1 626 042
- US-A1- 2008 299 016

## Description

### Field of the invention

The present invention refers to integrated ammonia-urea processes and related plants.

### Prior Art

Urea is synthesized by reacting ammonia and carbon dioxide. Both the ammonia feed and the carbon dioxide feed of a urea plant can be produced in an ammonia plant and, hence, integration of urea / ammonia plants is a known practice. In addition, an ammonia plant is a heat producer and a urea plant is a heat consumer, this being another incentive for integration. Typically, the heat produced by the ammonia plant does not cover the need of the overall ammonia-urea plant, which means that an additional heat input is necessary. This additional heat input is normally produced by burning fuel in auxiliary burners.

An ammonia-urea plant comprises an ammonia section and a urea section. In the ammonia section, ammonia is synthesized from hydrogen and nitrogen at a suitable high pressure and in the presence of a catalyst; hydrogen usually comes from reforming of a hydrocarbon source, such as natural gas, and nitrogen usually comes from air, e.g. from oxidant air which enters a secondary reformer. The reforming of the hydrocarbon source takes place in a front-end of the ammonia section, which includes a CO₂-removal unit for purification of the make-up gas; carbon dioxide extracted from said unit may feed the urea section.

The urea plant includes typically a high-pressure synthesis section, a recovery section at a medium and/or low pressure, and a finishing section. A discussion of the various processes and related plants for production of urea can be found in the literature, e.g. Ullmann's Encyclopedia of Industrial Chemistry, Wiley-VCH Verlag.

All the known urea processes generate also a purge gas stream. This purge gas stream is taken to remove inerts (such as nitrogen, hydrogen and oxygen) and avoid their accumulation in the urea plant. Said purge gas stream contains a certain amount of gaseous ammonia and causes an environmental concern if discharged in atmosphere as such.

It has been proposed in the prior art to recycle the urea purge gas stream as a feedstock for the integrated ammonia process. For example, EP 0 748 794 discloses to combine a high pressure urea synthesis purge stream with an oxidant feed of an autothermal reformer in the ammonia section.

However, another environmental concern is caused by combustion flue gas emitted by the ammonia-urea plant, typically by the ammonia section. Said flue gas may include fumes from the primary reformer of the front-end of the ammonia-section, and possibly fumes from boilers for generation of additional heat (steam) for the urea plant. Said fumes contain pollutants including, in particular, nitrogen oxides (NOx), and their purification is an additional cost. Using the urea purge gas stream as a fuel for the ammonia section provides a recycle of ammonia, but does not provide a solution to the problem of nitrogen oxides. Actually, the nitrogen oxides can be increased when using the purge gas stream as a fuel.

### Summary of the invention

It is proposed to use the NH₃-containing urea synthesis purge gas stream as an ammonia source for selective catalytic reduction of nitrogen oxides (NO_{X}) contained in the combustion fumes generated by the ammonia section.

Hence, the present invention provides a process where ammonia is synthesized in an ammonia section of an ammonia-urea integrated plant by reacting hydrogen and nitrogen, said hydrogen being produced by reforming of a hydrocarbon source; at least part of said ammonia is used for the synthesis of urea in a urea section of said plant, and a purge gas stream containing some ammonia is generated in said urea section, the process being characterized in that said purge gas stream is used as an ammonia source for selective catalytic reduction of nitrogen oxides contained in combustion fumes produced by said ammonia-urea plant.

Accordingly, an aspect of the invention is an ammonia-urea plant including an ammonia section and a urea section, wherein ammonia is synthesized in the ammonia section from hydrogen and nitrogen, said hydrogen being obtained via reforming of a hydrocarbon source, and at least part of said ammonia is passed to the urea section for the synthesis of urea, characterized by comprising a selective catalytic reduction unit for removal of nitrogen oxides from combustion fumes produced in the plant, and by comprising a line arranged to feed a purge gas stream generated in the urea section to said unit, for use as ammonia source for said unit.

Another aspect of the invention is the modification of an existing ammonia-urea integrated plant, where a selective catalytic reduction unit is installed for removal of nitrogen oxides from combustion fumes produced in the plant, and a purge gas stream from the urea section of the plant is directed to said unit, or mixed with combustion fumes before entering said unit.

Selective catalytic reduction (SCR) of nitrogen oxides is a known process and need not be described in detail. Basically, the NOx reduction reaction takes place as the gases pass through a catalyst chamber mixed with ammonia. The reaction converts ammonia and nitrogen oxides (mainly NO and NO₂) into N₂ and water vapour.

The combustion fumes may be emitted by the ammonia section, in particular by the primary reformer and/or secondary reformer in the front-end, and additional boilers fed with natural gas or another fuel. All the purge gas taken from the urea section, or a part thereof, can be used in the SCR process. According to different embodiments, another ammonia source for the SCR process can be provided, if necessary.

The advantage of the invention is the internal re-use of the purge gas stream generated by the urea section and, at the same time, the reduction of the pollutants nitrogen oxides in the flue gas produced in the ammonia section. A noticeable advantage of the invention is the avoidance of discharge into the atmosphere of ammonia and of nitrogen oxides as well.

### Description of a preferred embodiment

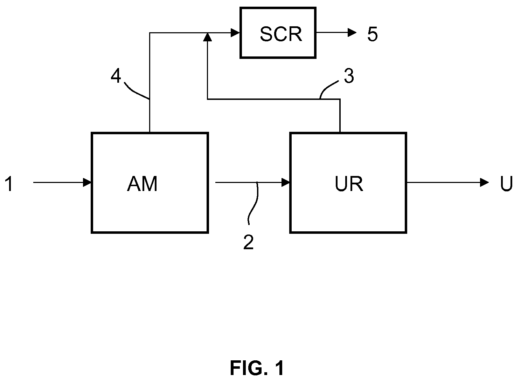

Fig. 1 shows a block scheme of an embodiment of the invention.

An integrated ammonia-urea plant comprises an ammonia section AM and a urea section UR.

The ammonia section AM receives a feed 1 of a suitable hydrocarbon source, such as natural gas, or a feedstock containing methane, or another source, for example naphta. Said ammonia section AM comprises a front-end for conversion of the feed 1 and air into a suitable make-up gas, and a synthesis loop. The front end may comprise a primary reformer, a secondary reformer, a CO-shift converter, a CO₂ removal unit and a methanator according to known art. A typical front-end of an ammonia plant is described for example in EP 2 022 754.

Ammonia 2 produced in the section AM is passed to the urea section UR, where urea U is synthesized. According to different embodiments, all the ammonia produced in section AM, or a part thereof, can be passed to the urea section UR. The urea section UR can implement any of the known processes for synthesis of urea, including e.g. the CO₂ stripping process, the self-stripping process or another. The urea section UR is advantageously fed also with carbon dioxide coming from purification of the make-up gas in the front-end of the ammonia section AM.

A purge gas stream 3, which contains some ammonia, is vented from the urea section UR. This purge gas stream 3, or at least a portion thereof, is directed to a selective catalytic reduction unit SCR for treatment of combustion fumes 4 from the ammonia section AM. In this example, the purge gas stream 3 is mixed with the fumes 4 before the fumes enter the unit SCR. In other embodiments, the stream 3 may be fed to the unit SCR. Said purge gas stream 3 provides ammonia for reduction of the nitrogen oxides in the fumes 4; purified flue gas 5 discharged to atmosphere contains mainly N₂ and H₂O and has a reduced or even negligible content of nitrogen oxides. Another source of ammonia can be provided for the unit SCR, whenever appropriate.

## Claims

1. A process where: ammonia (2) is synthesized in an ammonia section (AM) of an ammonia-urea integrated plant by reacting hydrogen and nitrogen, said hydrogen being produced by reforming of a hydrocarbon source (1); at least part of said ammonia (2) is reacted in a urea section (UR) of said plant for the synthesis of urea (U), and a purge gas stream (3) containing some ammonia is generated by said urea section, the process being **characterized in that** said purge gas stream (3) is used as an ammonia source for selective catalytic reduction of nitrogen oxides contained in combustion fumes (4) produced in the ammonia-urea plant.

2. A process according to claim 1, where said combustion fumes (4) are emitted by the ammonia section (AM) of said ammonia-urea plant.

3. A process according to claim 1 or 2, wherein said purge gas stream (3), or at least a portion thereof, is fed to a selective catalytic reduction unit (SCR) or is mixed with said combustion fumes (4) before entering said reduction unit.

4. An ammonia-urea plant including an ammonia section (AM) and a urea section (UR), wherein ammonia (2) is synthesized in the ammonia section from hydrogen and nitrogen, said hydrogen being obtained via reforming of a hydrocarbon source (1), and at least part of said ammonia (2) is passed to the urea section for the synthesis of urea, said plant being **characterized by** comprising a selective catalytic reduction unit (SCR) for removal of nitrogen oxides from combustion fumes (4) produced in the plant, and by comprising a line arranged to feed a purge gas stream (3) generated by the urea section to said selective catalytic reduction unit (SCR), for use of said purge gas stream (3) as an ammonia source for said unit.

5. A method for the modification of an existing ammonia-urea integrated plant, comprising: the installation of a selective catalytic reduction unit (SCR) for removal of nitrogen oxides from combustion fumes (4) produced in the plant, and the provision of a purge gas line (3) which is arranged to direct a purge gas stream generated from the urea section to said selective catalytic reduction unit (SCR), or to mix said purge gas stream with a flow of combustion fumes entering said reduction unit.

## Patentansprüche

1. Verfahren, bei dem: Ammoniak (2) in einem Ammoniakbereich (AM) einer integrierten Ammoniak-Harnstoff-Anlage synthetisiert wird durch Reagieren von Wasserstoff und Stickstoff, wobei der Wasserstoff durch Reformieren einer Kohlenwasserstoffquelle (1) erzeugt wird; zumindest ein Teil des Ammoniaks (2) in einem Harnstoffbereich (UR) der Anlage zur Synthese von Harnstoff (U) reagiert wird und ein Spülgasstrom (3), der etwas Ammoniak enthält, durch den Harnstoffbereich erzeugt wird,
wobei das Verfahren **dadurch gekennzeichnet ist, dass** der Spülgasstrom (3) als Ammoniakquelle zur selektiven katalytischen Reduktion von Stickoxiden verwendet wird, die in Verbrennungsdämpfen (4) enthalten sind, die in der Ammoniak-Harnstoff-Anlage erzeugt werden.

2. Verfahren nach Anspruch 1,
wobei die Verbrennungsdämpfe (4) von dem Ammoniakbereich (AM) der Ammoniak-Harnstoff-Anlage emittiert werden.

3. Verfahren nach Anspruch 1 oder 2,
wobei der Spülgasstrom (3) oder zumindest ein Teil davon zu einer selektiven katalytischen Reduktionseinheit (SCR) geführt wird oder vor dem Eintritt in die Reduktionseinheit mit den Verbrennungsdämpfen (4) gemischt wird.

4. Ammoniak-Harnstoff-Anlage mit einem Ammoniakbereich (AM) und einem Harnstoffbereich (UR), wobei Ammoniak (2) in dem Ammoniakbereich aus Wasserstoff und Stickstoff synthetisiert wird, wobei der Wasserstoff durch Reformieren einer Kohlenwasserstoffquelle (1) gebildet wird und wobei zumindest ein Teil des Ammoniaks (2) für die Synthese von Harnstoff zu dem Harnstoffbereich geleitet wird,
wobei die Anlage **dadurch gekennzeichnet ist, dass** sie eine selektive katalytische Reduktionseinheit (SCR) zum Entfernen von Stickoxiden aus Verbrennungsdämpfen (4) aufweist, die in der Anlage erzeugt werden, und dass die eine Leitung aufweist, die zum Zuführen eines von dem Harnstoffbereich erzeugten Spülgasstroms (3) zu der selektiven katalytischen Reduktionseinheit (SCR) ausgebildet ist, um den Spülgasstrom (3) als Ammoniakquelle für die Einheit zu nutzen.

5. Verfahren zum Modifizieren einer bestehenden integrierten Ammoniak-Harnstoff-Anlage, das folgende Schritte aufweist: den Einbau einer selektiven katalytischen Reduktionseinheit (SCR) zum Entfernen von Stickoxiden aus in der Anlage erzeugten Verbrennungsdämpfen (4), sowie die Bereitstellung einer Spülgasleitung (3), die dazu ausgebildet ist, einen von dem Harnstoffbereich erzeugten Spülgasstrom zu der selektiven katalytischen Reduktionseinheit (SCR) zu leiten oder den Spülgasstrom mit einer Strömung von in die Reduktionseinheit eintretenden Verbrennungsdämpfen zu mischen.

## Revendications

1. Un procédé où : de l'ammoniac (2) est synthétisé dans une section de production d'ammoniac (AM) d'une usine intégrée d'ammoniac-urée en faisant réagir de l'hydrogène et de l'azote, ledit hydrogène étant issu du reformage d'une source d'hydrocarbures (1) ; au moins une partie dudit ammoniac (2) est mise en réaction dans une section de production d'urée (UR) de ladite usine afin de synthétiser l'urée (U), et un courant de gaz de purge (3) contenant de l'ammoniac est généré par ladite section de production d'urée, le procédé étant **caractérisé en ce que** le courant de gaz de purge (3) est utilisé comme source d'ammoniac pour la réduction catalytique sélective d'oxydes d'azote contenus dans des fumées de combustion (4) émises par l'usine d'ammoniac-urée.

2. Un procédé selon la revendication 1, dans lequel lesdites fumées de combustion (4) sont émises par la section de production d'ammoniac (AM) de ladite usine d'ammoniac-urée.

3. Un procédé selon la revendication 1 ou 2, dans lequel ledit courant de gaz de purge (3), ou au moins une partie de celui-ci, est acheminé vers une unité de réduction catalytique sélective (RCS) ou est mélangé avec lesdites fumées de combustion (4) avant d'être introduit dans ladite unité de réduction.

4. Une usine d'ammoniac-urée comportant une section de production d'ammoniac (AM) et une section de production d'urée (UR), dans laquelle l'ammoniac (2) est synthétisé dans la section de production d'ammoniac à partir d'hydrogène et d'azote, ledit hydrogène étant issu du reformage d'une source d'hydrocarbures (1), et au moins une partie dudit ammoniac (2) est envoyée à la section de production d'urée afin de synthétiser l'urée, ladite usine étant **caractérisée en ce qu'**elle comprend une unité de réduction catalytique sélective (RCS) afin d'éliminer les oxydes d'azote contenus dans les fumées de combustion (4) émises par l'usine, et **en ce qu'**elle comprend une ligne conçue pour acheminer un courant de gaz de purge (3) généré par la section de production d'urée vers ladite unité de réduction catalytique sélective (RCS), afin d'utiliser ledit courant de gaz de purge (3) comme source d'ammoniac pour ladite unité.

5. Une méthode de modification d'une usine intégrée d'ammoniac-urée existante, comprenant : l'installation d'une unité de réduction catalytique sélective (RCS) afin d'éliminer les oxydes d'azote contenus dans les fumées de combustion (4) émises par l'usine, et la fourniture d'une ligne de gaz de purge (3) conçue pour acheminer un courant de gaz de purge généré par la section de production d'urée vers ladite unité de réduction catalytique sélective (RCS), ou pour mélanger ledit courant de gaz de purge avec un flux de fumées de combustion introduit dans ladite unité de réduction.
